# EUROPEAN PATENT SPECIFICATION

(11) **EP 3 803 398 B1**
(45) Date of publication and mention of the grant of the patent: **13.08.2025**
(21) Application number: 19815519.4
(22) Date of filing: 07.06.2019
(51) Int. Cl.: G01N 33/53, G01N 33/543, C07H 21/00, C12Q 1/6832

(54) **MODIFIED NUCLEOBASES WITH UNIFORM H-BONDING INTERACTIONS, HOMO- AND HETERO-BASEPAIR BIAS, AND MISMATCH DISCRIMINATION**
MODIFIZIERTE NUKLEOBASEN MIT GLEICHFÖRMIGEN H-BINDUNGSWECHSELWIRKUNGEN, HOMO- UND HETERO-BASENPAARVERZERRUNG UND UNGLEICHGEWICHTSUNTERSCHEIDUNG
NUCLÉOBASES MODIFIÉES AYANT DES INTERACTIONS DE LIAISON H UNIFORMES, UNE POLARISATION D'HOMO-ET HÉTÉRO-BASE, ET UNE DISCRIMINATION DE MÉSAPPARIEMENTS

(30) Priority: 08.06.2018 US 201862763299 P
(43) Date of publication of application: 14.04.2021
(73) Proprietor: CARNEGIE MELLON UNIVERSITY, Pittsburgh, Pennsylvania 15213 (US)
(72) Inventor: LY, Danith, H., Pittsburgh, Pennsylvania 15243 (US); THADKE, Shivaji, A., Pittsburgh, Pennsylvania 15217 (US); SHAIKH, Ashif, Y., Pune, Maharashtra 413115 Baramati (IN); HSIEH, Wei-Che, New Taipei City, 234 (TW); NAKHI, Ali, Falcon Heights, Minnesota 55108 (US); PERERA, J. Dinithi, Pittsburgh, Pennsylvania 15217 (US)
(74) Representative: Grünecker Patent- und Rechtsanwälte PartG mbB
(86) International application number: PCT/US2019/036017
(87) International publication number: WO 2019/236979

(56) References cited:
- WO-A1-2018/058091
- US-A1- 2016 264 614
- US-A1- 2017 136 131
- GUPTA PANKAJ ET AL: "Triple helical recognition of pyrimidine inversions in polypurine tracts of RNA by nucleobase-modified PNA", CHEMICAL COMMUNICATIONS, vol. 47, no. 39, 1 January 2011 (2011-01-01), UK, pages 11125, XP055887492, ISSN: 1359-7345, DOI: 10.1039/c1cc14706d
- ST. AMANT ANDRÉ H. ET AL: "Synthesis and oligomerization of Fmoc/Boc-protected PNA monomers of 2,6-diaminopurine, 2-aminopurine and thymine", ORGANIC & BIOMOLECULAR CHEMISTRY, vol. 10, no. 4, 1 January 2012 (2012-01-01), pages 876 - 881, XP055887512, ISSN: 1477-0520, DOI: 10.1039/C1OB06582C
- SANDERS JEFFREY M. ET AL: "Effects of Hypoxanthine Substitution in Peptide Nucleic Acids Targeting KRAS2 Oncogenic mRNA Molecules: Theory and Experiment", JOURNAL OF PHYSICAL CHEMISTRY PART B, vol. 117, no. 39, 18 September 2013 (2013-09-18), US, pages 11584 - 11595, XP055887516, ISSN: 1520-6106, DOI: 10.1021/jp4064966
- BICHISMITA SAHU ET AL: "Synthesis of Conformationally Preorganized and Cell-Permeable Guanidine-Based γ-Peptide Nucleic Acids (γGPNAs)", THE JOURNAL OF ORGANIC CHEMISTRY, vol. 74, no. 4, 20 February 2009 (2009-02-20), pages 1509 - 1516, XP055187182, ISSN: 0022-3263, DOI: 10.1021/jo802211n
- WAGNER T ET AL: "Synthesis of amino acids with unnatural nucleobases or chromophores suitable for use in model electron-transfer studies", EUROPEAN JOURNAL OF ORGANIC CHEMISTRY, WILEY-VCH, DE, 1 January 2003 (2003-01-01), pages 3673 - 3679, XP002408149, ISSN: 1434-193X, DOI: 10.1002/EJOC.200300242
- TAURAITE ET AL.: "Modified Nucleotides as Substrates of Terminal Deoxynucleotidyl Transferase", MOLECULES, vol. 22, no. 4, 22 April 2017 (2017-04-22), pages 672, XP055489993

## Description

### BACKGROUND

Described herein are nucleobases, polymer monomers comprising the nucleobases and nucleic acids and analogs thereof comprising the nucleobases. Also described herein are methods of use of the nucleobases, polymer monomers comprising the nucleobases and nucleic acids and analogs thereof comprising the nucleobases (not part of the claimed invention).

For most organisms, genetic information is encoded in double-stranded DNA in the form of Watson-Crick base-pairing-in which adenine (A) pairs with thymine (T) and cytosine (C) with guanine (G). Depending on which set of this genetic information is decoded through transcription and translation, the developmental program and physiological status will be determined. Development of molecules that can be tailor-designed to bind sequence-specifically to any part of this genetic biopolymer (DNA or RNA), thereby enabling the control of the flow of genetic information and assessment and manipulation of the genome's structures and functions, is important for biological and biomedical research in the effort to unravel the molecular basis of life, including molecular tools for basic research in biology. This effort is also important for medicinal and therapeutic applications for the treatment and detection of genetic diseases.

Oligonucleotides are versatile in their use as molecular tools for basic research in biology, as well as molecular reagents for therapeutic and diagnostics applications, e.g., in the treatment and detection of genetic diseases. Generally, oligonucleotide molecules are short pieces (10-30 nucleotides in length) of single-stranded DNA or RNA, or derivatives thereof. They may include sugar phosphodiester backbones, or other backbones, that are connected to the adenine (A), cytosine (C), guanine (G), and thymine (T) or uridine (U) nucleobases. They are designed to bind to the DNA or RNA targets through Watson-Crick base-pairing in which A pairs with T (or U) and C with G. Oligonucleotide molecules have been employed in a broad range of applications, including, for example, interrogation of nucleic acid sequence information, manipulation of RNA structure, and regulation of gene expression. The success of many of these applications rely on the ability of the oligomers to bind to DNA or RNA targets in a tight and sequence-specific manner. Additional requirements for intracellular and in vivo gene targeting include enzymatic stability and cell permeability.

The document to Gupta et al. (Chemical Communications, 2011, Vol. 47, No. 39, pp. 11125) relates to peptide nucleic acids containing 2-pyrimidinone and 3-oxo-2,3-dihydropyridazine heterocycles.

The document to St. Amant et al. (Organic & Biomolecular Chemistry, 2012, Vol. 10, No. 4, pp. 876-881) relates to a Boc-protecting group strategy for Fmoc-based peptide nucleic acid oligomerization for thymine, 2,6-diaminopurine, and 2-aminopurine.

The document to Sanders et al. (Journal of Physical Chemistry Part B, 2013, Vol. 117, No. 39, pp. 11584-11595) relates a polynucleotide comprising a hypoxanthine nucleobase attached via a peptide nucleotide backbone and RNA duplexes thereof.

The document to Sahu et al. (The Journal of Organic Chemistry, 2009, Vol. 74, No. 4, pp. 1509-1516) relates to a method of preparing optically-pure guanidine-based γ-peptide nucleic acid monomers for all four natural nucleobases (A, C, G, and T).

The document WO 2018/058091 A1 relates to divalent nucleobases that each bind two nucleic acid strands, matched or mismatched when incorporated into a nucleic acid or nucleic acid analog backbone.

The document to Wagner et al. (European Journal of Organic Chemistry, 2003, pp. 3673-2679) relates to a method of preparing Boc-protected amino acids bearing chromophores, as well as nucleo amino acids with artificial purines.

### SUMMARY

The present invention is defined in the appended claims.

A genetic recognition reagent is provided, comprising a plurality of nucleobase moieties attached to a nucleic acid or nucleic acid analog backbone, in which at least one nucleobase moiety is: 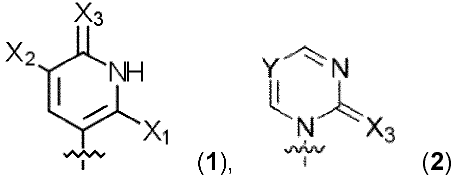 (not part of the claimed invention), 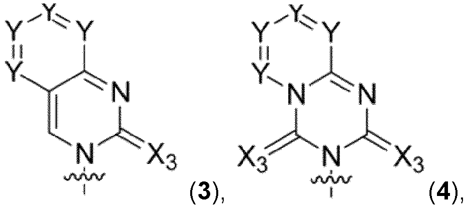 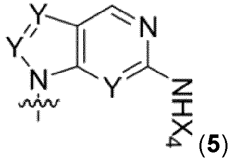 (not part of the claimed invention), or 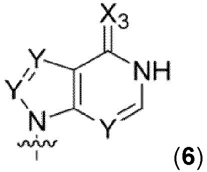 (not part of the claimed invention),
wherein, X₁ is =O (= referring to a double bond) (not part of the claimed invention), =S (not part of the claimed invention), =Se (not part of the claimed invention), or CH₃; X₂ is H, CH₃, CN, NC, N₃, C(O)OH, or C(O)NH₂; X₃ is O or S; X₄ (not part of the claimed invention) is H, C(O)CH₃, or C(O)OCH₃; and Y is N or CH, wherein in (1), when X₁ and X₃ are O, X₂ is not H or methyl (not part of the claimed invention).

A compound also is provided (not part of the claimed invention), comprising a nucleic acid backbone monomer or nucleic acid analog backbone monomer linked to a nucleobase moiety having the structure: 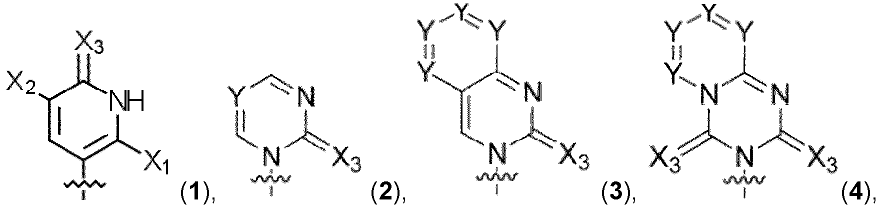 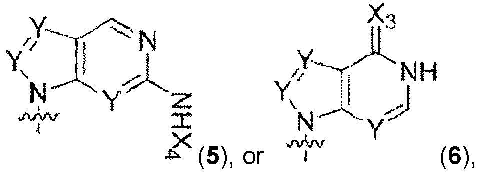 wherein, X₁ is =O, =S, =Se, or CH₃; X₂ is H, CH₃, CN, NC, N₃, C(O)OH, or C(O)NH₂; X₃ is O or S; X₄ is H, C(O)CH₃, or C(O)OCH₃; and Y is N or CH, wherein in (I), when X₁ and X₃ are O, X₂ is not H or methyl.

### BRIEF DESCRIPTION OF THE DRAWINGS

Figure 1 illustrates the hydrogen-bonding interactions of (A) modified vs. modified nucleobases (homoduplex), (B) natural vs. natural, (C) modified vs. natural (heteroduplex).

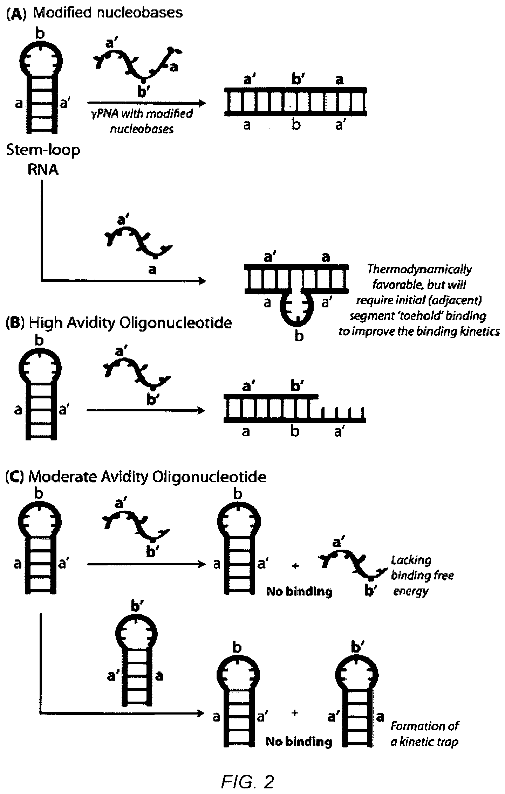

Figure 2 is schematic diagrams showing a distinct advantage that the newly designed oligonucleotide molecules provide in being able to selectively target RNA secondary structure. (A) Despite the (a'/a) sequence complementarity, a PNA oligomer (chiral or achiral) containing modified (u, c, a, and g) nucleobases cannot adopt a hairpin structure and is able to hybridize to its complementary stem-loop RNA target. (B) Tight-binding oligonucleotide molecule such as LNA or yPNA is able to invade the stem-loop structure, but its application in therapeutics and diagnostics poses considerable risks due to the nonspecific binding. (C) Moderate avidity oligonucleotides, a category in which most oligonucleotide molecules fall under, are unable to open the stem-loop structure due to the lack of binding free energy, or as the result of the formation of a kinetic (hairpin) trap.
Figure 3 schematically depicts examples of RNA (A) secondary and (B) tertiary structures that oligonucleotide molecules described herein are able to selectively target that would otherwise be difficult to accomplish with existing nucleic acid systems.
Figure 4 provides structures of exemplary nucleobases.
Figure 5 depicts exemplary nucleic acid analog residues for nucleic acid analogs, including: phosphorothioate DNA (PS DNA), α, β-constrained nucleic acid (α,β-CNA), 2'-methoxyl RNA, 2'-fluoro RNA, phosphorodiamidate morpholino oligomer (PMO), locked nucleic acid (LNA), 2',4'-constrained ethyl nucleic acid ((S)-cEt), 2',4' bridged nucleic acid NC (N-H) (BNA-NC(N-H)), 2',4' bridged nucleic acid NC (N-methyl) (BNA-NC(N-Me)), ((S)-5'-C-methyl DNA (RNA)), and 5'-E-vinylphosphonate nucleic acid (E-VP), wherein R is H, OH, F, OMe, or O(CH₂)₂OMe.
Figure 6 provides exemplary synthetic schemes for selected nucleobases.
Figure 7 provides an exemplary synthetic scheme for selected cell-permeable γPNA monomers.

### DETAILED DESCRIPTION

The use of numerical values in the various ranges specified in this application, unless expressly indicated otherwise, are stated as approximations as though the minimum and maximum values within the stated ranges are both preceded by the word "about". In this manner, slight variations above and below the stated ranges can be used to achieve substantially the same results as values within the ranges. Also, unless indicated otherwise, the disclosure of ranges is intended as a continuous range including every value between the minimum and maximum values. As used herein "a" and "an" refer to one or more.

As used herein, the term "comprising" is open-ended and can be synonymous with "including", "containing", or "characterized by". As used herein, embodiments "comprising" one or more stated elements or steps also include, but are not limited to embodiments "consisting essentially of" and "consisting of" these stated elements or steps.

The term "polymer composition" is a composition comprising one or more polymers. As a class, "polymers" include, without limitation, homopolymers, heteropolymers, co-polymers, block polymers, block co-polymers and can be both natural and synthetic. Homopolymers contain one type of building block, or monomer, whereas co-polymers contain more than one type of monomer. An "oligomer" is a polymer that comprises a small number of monomers, such as, for example, from 3 to 100 monomer residues. As such, the term "polymer" includes oligomers. The terms "nucleic acid" and "nucleic acid analog" includes nucleic acid and nucleic acid polymers and oligomers.

A polymer "comprises" or is "derived from" a stated monomer if that monomer is incorporated into the polymer. Thus, the incorporated monomer that the polymer comprises is not the same as the monomer prior to incorporation into a polymer, in that at the very least, certain linking groups are incorporated into the polymer backbone or certain groups are removed in the polymerization process. A polymer is said to comprise a specific type of linkage if that linkage is present in the polymer. An incorporated monomer is a "residue". A typical monomer for a nucleic acid or nucleic acid analog is referred to as a nucleotide or a nucleotide residue when incorporated into a polymer.

A "moiety" (*pl.* "moieties") is a part of a chemical compound, and comprises groups, such as functional groups. As such, a nucleobase moiety is a nucleobase that is modified by attachment to another compound moiety, such as a polymer monomer, e.g. the nucleic acid or nucleic acid analog monomers described herein, or a polymer, such as a nucleic acid or nucleic acid analog as described herein.

"Alkyl" refers to straight, branched chain, or cyclic hydrocarbon groups including from 1 to about 20 carbon atoms, for example and without limitation C₁₋₃, C₁₋₆, C₁₋₁₀ groups, for example and without limitation, straight, branched chain alkyl groups such as methyl, ethyl, propyl, butyl, pentyl, hexyl, heptyl, octyl, nonyl, decyl, undecyl, dodecyl, and the like. An alkyl group can be, for example, a C₁, C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, C₂₄, C₂₅, C₂₆, C₂₇, C₂₈, C₂₉, C₃₀, C₃₁, C₃₂, C₃₃, C₃₄, C₃₅, C₃₆, C₃₇, C₃₈, C₃₉, C₄₀, C₄₁, C₄₂, C₄₃, C₄₄, C₄₅, C₄₆, C₄₇, C₄₈, C₄₉, or C₅₀ group that is substituted or unsubstituted. Non-limiting examples of straight alkyl groups include methyl, ethyl, propyl, butyl, pentyl, hexyl, heptyl, octyl, nonyl, and decyl. Branched alkyl groups comprises any straight alkyl group substituted with any number of alkyl groups. Non-limiting examples of branched alkyl groups include isopropyl, isobutyl, sec-butyl, and t-butyl. Non-limiting examples of cyclic alkyl groups include cyclopropyl, cyclobutyl, cyclopentyl, cyclohexyl, cycloheptlyl, and cyclooctyl groups. Cyclic alkyl groups also comprise fused-, bridged-, and spiro-bicycles and higher fused-, bridged-, and spiro-systems. A cyclic alkyl group can be substituted with any number of straight, branched, or cyclic alkyl groups.

"Substituted alkyl" refers to alkyl substituted at 1 or more, (e.g., 1, 2, 3, 4, 5, or 6) positions, which substituents are attached at any available atom to produce a stable compound, with substitution as described herein. "Optionally substituted alkyl" refers to alkyl or substituted alkyl. "Halogen," "halide," and "halo" refers to -F, -Cl, -Br, and/or -I. "Alkylene" and "substituted alkylene" refer to divalent alkyl and divalent substituted alkyl, respectively, including, without limitation, ethylene (-CH₂-CH₂-). "Optionally substituted alkylene" refers to alkylene or substituted alkylene.

"Alkene or alkenyl" refers to straight, branched chain, or cyclic hydrocarbyl groups including from 2 to about 20 carbon atoms, such as, without limitation C₂₋₃, C₂₋₆, C₂₋₁₀ groups having one or more (e.g., 1, 2, 3, 4, or 5) carbon-to-carbon double bonds. The olefin or olefins of an alkenyl group can be, for example, *E, Z, cis, trans,* terminal, or exo-methylene. An alkenyl or alkenylene group can be, for example, a C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, C₂₄, C₂₅, C₂₆, C₂₇, C₂₈, C₂₉, C₃₀, C₃₁, C₃₂, C₃₃, C₃₄, C₃₅, C₃₆, C₃₇, C₃₈, C₃₉, C₄₀, C₄₁, C₄₂, C₄₃, C₄₄, C₄₅, C₄₆, C₄₇, C₄₈, C₄₉, or C₅₀ group that is substituted or unsubstituted. A halo-alkenyl group can be any alkenyl group substituted with any number of halogen atoms.

"Substituted alkene" refers to alkene substituted at 1 or more (e.g., 1, 2, 3, 4, or 5 positions) which substituents are attached at any available atom to produce a stable compound, with substitution as described herein. "Optionally substituted alkene" refers to alkene or substituted alkene. Likewise, "alkenylene" refers to divalent alkene. Examples of alkenylene include without limitation, ethenylene (-CH=CH-) and all stereoisomeric and conformational isomeric forms thereof. "Substituted alkenylene" refers to divalent substituted alkene. "Optionally substituted alkenylene" refers to alkenylene or substituted alkenylene.

Alkyne or "alkynyl" refers to a straight, branched chain, or cyclic unsaturated hydrocarbon having the indicated number of carbon atoms and at least one triple bond. The triple bond of an alkyne or alkynyl group can be internal or terminal. Examples of a (C₂-C₈)alkynyl group include, but are not limited to, acetylene, propyne, 1-butyne, 2-butyne, 1- pentyne, 2-pentyne, 1-hexyne, 2-hexyne, 3-hexyne, 1-heptyne, 2-heptyne, 3-heptyne, 1-octyne, 2-octyne, 3-octyne and 4-octyne. An alkynyl group can be unsubstituted or optionally substituted with one or more substituents as described herein below. An alkyne or alkynyl group can be, for example, a C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, C₂₄, C₂₅, C₂₆, C₂₇, C₂₈, C₂₉, C₃₀, C₃₁, C₃₂, C₃₃, C₃₄, C₃₅, C₃₆, C₃₇, C₃₈, C₃₉, C₄₀, C₄₁, C₄₂, C₄₃, C₄₄, C₄₅, C₄₆, C₄₇, C₄₈, C₄₉, or C₅₀ group that is substituted or unsubstituted. A halo-alkynyl group can be any alkynyl group substituted with any number of halogen atoms.

The term "alkynylene" refers to divalent alkyne. Examples of alkynylene include without limitation, ethynylene, propynylene. "Substituted alkynylene" refers to divalent substituted alkyne.

The term "alkoxy" refers to an -O-alkyl group having the indicated number of carbon atoms. An ether or an ether group comprises an alkoxy group. For example, a (C₁-C₆)alkoxy group includes -O-methyl (methoxy), -O-ethyl (ethoxy), -O-propyl (propoxy), -O-isopropyl (isopropoxy), -O-butyl (butoxy), -O-sec -butyl (sec-butoxy), - O-tert-butyl (tert-butoxy), -O-pentyl (pentoxy), -O-isopentyl (isopentoxy), -O-neopentyl (neopentoxy), -O-hexyl (hexyloxy), -O-isohexyl (isohexyloxy), and -O-neohexyl (neohexyloxy). "Hydroxyalkyl" refers to a (C₁-C₁₀)alkyl group wherein one or more of the alkyl group's hydrogen atoms is replaced with an -OH group. Examples of hydroxyalkyl groups include, but are not limited to, -CH₂OH, -CH₂CH₂OH, - CH₂CH₂CH₂OH, -CH₂CH₂CH₂CH₂OH, -CH₂CH₂CH₂CH₂CH₂OH, - CH₂CH₂CH₂CH₂CH₂CH₂OH, and branched versions thereof. The term "ether" or "oxygen ether" refers to (C₁-C₁₀)alkyl group wherein one or more of the alkyl group's carbon atoms is replaced with an -O- group. The term ether includes -CH₂-(OCH₂-CH₂)_{q}OP₁ compounds where P₁ is a protecting group, -H, or a (C₁-C₁₀)alkyl. Exemplary ethers include polyethylene glycol, diethylether, methylhexyl ether and the like.

The term "thioether" refers to (C₁-C₁₀)alkyl group wherein one or more of the alkyl group's carbon atoms is replaced with an -S- group. The term thioether includes -CH₂-(SCH₂-CH₂)_{q}-SP₁ compounds where P₁ is a protecting group, -H, or a (C₁-C₁₀)alkyl. Exemplary thioethers include dimethylthioether or ethylmethyl thioether.

Protecting groups (e.g., for protecting amines during synthesis of compounds described herein) are known in the art and include, without limitation: 9-fluorenylmethyloxy carbonyl (Fmoc), t-butyloxycarbonyl (Boc), benzhydryloxycarbonyl (Bhoc), benzyloxycarbonyl (Cbz), O-nitroveratryloxycarbonyl (Nvoc), benzyl (Bn), allyloxycarbonyl (alloc), trityl (Trt), dimethoxytrityl (DMT), I-(4,4-dimethyl-2,6-dioxacyclohexylidene)ethyl (Dde), diathiasuccinoyl (Dts), benzothiazole-2-sulfonyl (Bts) and monomethoxytrityl (MMT) groups.

"Aryl," alone or in combination refers to an aromatic monocyclic or bicyclic ring system such as phenyl or naphthyl. "Aryl" also includes aromatic ring systems that are optionally fused with a cycloalkyl ring. A "substituted aryl" is an aryl that is independently substituted with one or more substituents attached at any available atom to produce a stable compound, wherein the substituents are as described herein. The substituents can be, for example, hydrocarbyl groups, alkyl groups, alkoxy groups, and halogen atoms. "Optionally substituted aryl" refers to aryl or substituted aryl. An aryloxy group can be, for example, an oxygen atom substituted with any aryl group, such as phenoxy. An arylalkoxy group can be, for example, an oxygen atom substituted with any aralkyl group, such as benzyloxy.

"Arylene" denotes divalent aryl, and "substituted arylene" refers to divalent substituted aryl. "Optionally substituted arylene" refers to arylene or substituted arylene.

"Heteroatom" refers to N, O, P and S. Compounds that contain N or S atoms can be optionally oxidized to the corresponding N-oxide, sulfoxide or sulfone compounds. "Hetero-substituted" refers to an organic compound in any embodiment described herein in which one or more carbon atoms are substituted with N, O, P or S.

"Cycloalkyl" refer to monocyclic, bicyclic, tricyclic, or polycyclic, 3- to 14-membered ring systems, which are either saturated, unsaturated or aromatic. The cycloalkyl group may be attached via any atom. Cycloalkyl also contemplates fused rings wherein the cycloalkyl is fused to an aryl or hetroaryl ring. Representative examples of cycloalkyl include, but are not limited to cyclopropyl, cyclobutyl, cyclopentyl, and cyclohexyl. A cycloalkyl group can be unsubstituted or optionally substituted with one or more substituents as described herein below. "Cycloalkylene" refers to divalent cycloalkyl. The term "optionally substituted cycloalkylene" refers to cycloalkylene that is substituted with 1, 2 or 3 substituents, attached at any available atom to produce a stable compound, wherein the substituents are as described herein.

"Carboxyl" or "carboxylic" refers to group having the indicated number of carbon atoms and terminating in a -C(O)OH group, thus having the structure -R-C(O)OH, where R is a divalent organic group that includes linear, branched, or cyclic hydrocarbons. Non-limiting examples of these include: C₁₋₈ carboxylic groups, such as ethanoic, propanoic, 2-methylpropanoic, butanoic, 2,2-dimethylpropanoic, pentanoic, *etc.*

"(C₃-C₈)aryl-(C₁-C₆)alkylene" refers to a divalent alkylene wherein one or more hydrogen atoms in the C₁-C₆ alkylene group is replaced by a (C₃-C₈)aryl group. Examples of (C₃-C₈)aryl-(C₁-C₆)alkylene groups include without limitation 1-phenylbutylene, phenyl-2-butylene, I-phenyl-2-methylpropylene, phenylmethylene, phenylpropylene, and naphthylethylene. The term "(C₃-C₈)cycloalkyl-(C₁-C₆)alkylene" refers to a divalent alkylene wherein one or more hydrogen atoms in the C₁-C₆ alkylene group is replaced by a (C₃-C₈)cycloalkyl group. Examples of (C₃-C₈)cycloalkyl-(C₁-C₆)alkylene groups include without limitation 1-cycloproylbutylene, cycloproyl-2-butylene, cyclopentyl-1 -phenyl-2-methylpropylene, cyclobutylmethylene and cyclohexylpropylene.

A moiety, such as a nucleobase moiety, functional group, guanidine-containing group, or PEG-containing group, in a larger molecule, such as a nucleic acid or nucleic acid polymer chain, e.g., as described herein, is "linked" to the remainder of the molecule, meaning it is covalently-attached either directly, or through an inert linking moiety, to the remainder of the molecule. By "inert," it is meant, that the linker does not substantially affect the function of the nucleic acid or nucleic acid analog for its intended use. Non-limiting examples of inert linkers include linear, branched, and/or cyclic hydrocarbyl or substituted hydrocarbyl moieties, for example having less than 15, 10, or 6 carbon atoms, such as an alkylene moiety, e.g., a methylene, ethylene, propylene, or butylene group, or an aryl group, and optionally containing a heteroatom, such an S (e.g., thioether), N (tertiary amine), or O (e.g., ether) atom, and/or a linking bond, such as, for example and without limitation, an ester, amide, carbamate, or carbonate linkage. A linker may serve as a spacer for physically or spatially separating two constituents of a molecule.

Provided herein are nucleic acids and analogs thereof, collectively "genetic recognition reagents", that bind specifically to a nucleic acid strand, e.g., under physiological conditions, e.g., in normal saline (0.9 % wt. NaCl), or another isotonic solution such as Tris-buffered saline or PBS, at 37°C. The genetic recognition reagent comprises a plurality of nucleobase moieties, each attached to a nucleic acid or nucleic acid analog backbone monomer residue, e.g., in the case of DNA or RNA, forming a nucleoside or, with the phosphate group, a nucleotide), and forming a part of the larger genetic recognition reagent comprising at least two nucleic acid or nucleic acid monomer residues, and therefore at least two nucleobase moieties.

In one aspect, all nucleobases of the genetic recognition reagents are modified nucleobases as described herein. In another embodiment, the genetic recognition reagents described herein comprise at least one modified nucleobase as described herein, with other nucleobases being natural nucleobases (e.g. adenine, guanine, cytosine, thymine, or uracil), or different from those modified bases.

Thus in one aspect, modified nucleobases are provided. Those nucleobases can be incorporated into a nucleic acid or nucleic acid analog monomer (e.g., nucleotide), which can then be incorporated into an oligomer or polymer of monomers with a desired sequence of nucleobases. Structures of the modified nucleobases are provided below, and include: 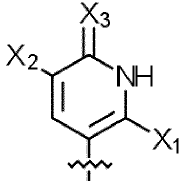 (not part of the claimed invention), 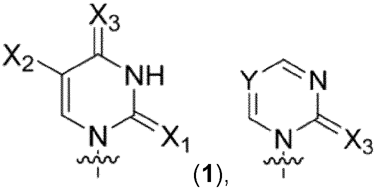 (2) (not part of the claimed invention), 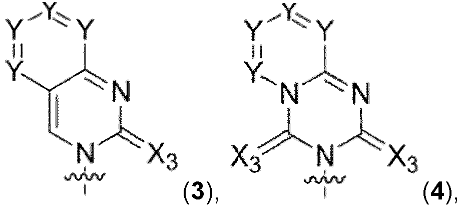 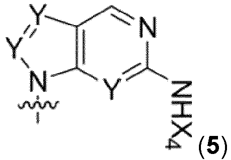 (not part of the claimed invention), or 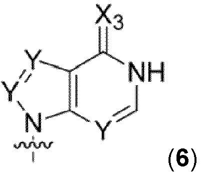 (not part of the claimed invention),
wherein, wherein,
X₁ is =O (= referring to a double bond) (not part of the claimed invention), =S (not part of the claimed invention), =Se (not part of the claimed invention), or CH₃;
X₂ is H, CH₃, CN, NC, N₃, C(O)OH, or C(O)NH₂;
X₃ is O or S;
X₄ (not part of the claimed invention) is H, C(O)CH₃, or C(O)OCH₃; and
Y is N or CH,
wherein in (1), when X₁ and X₃ are O, X₂ is not H or methyl (not part of the claimed invention). Non-limiting examples of such nucleobases, forming a complete set of nucleobases able to bind A, T, G, and C of natural DNA or RNA, include: 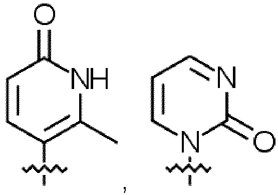 (not part of the claimed invention), 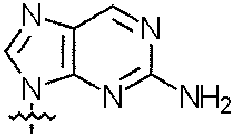 (not part of the claimed invention), or 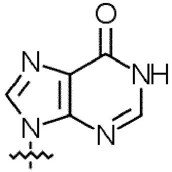 (not part of the claimed invention), Additional examples of such nucleobases include the fluorescent bases (not part of the claimed invention): 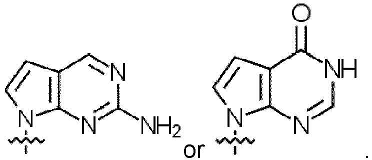 Any, some, or all of the preceding nucleobases may be incorporated into nucleotide monomers and a genetic recognition reagent.

In one aspect, the genetic recognition reagents described herein are capable of self-assembly on a nucleic acid template comprising a target sequence of the genetic recognition reagents. For example, a first genetic recognition reagent can hybridize to a first portion of a nucleic acid template. A second self-assembling genetic recognition reagent can hybridize to a second portion, adjacent to the first portion, of the nucleic acid template. The first genetic recognition reagent and the second genetic recognition reagent can either covalently-link or non-covalently-link together using various functional end groups to form a contiguous structure. The genetic recognition reagents can comprise a first moiety linked by a linker to the first end of the nucleic acid or nucleic acid analog backbone and a second moiety linked by a linker to the second end of the nucleic acid or nucleic acid analog backbone. The second moiety can be the same as the first moiety. The second moiety can be different than the first moiety. In one example, International Patent Application No. PCT/US18/67096 discloses self-assembling genetic recognition reagents that comprise aryl groups at their ends that pi-stack to form strong non-covalent linkages between unit genetic recognition reagents when aligned contiguously on a target sequence, for example an expanded repeat, such as TTC, TTCTTC, TCT, TCTTCT, CTT, CTTCTT, CCG, CCGCCG, CGC, CGCCGC, GCC, GCCGCC, CGG, CGGCGG, GCG, GCGGCG, GGC, GGCGGC, CTG, CTGCTG, TGC, TGCTGC, GCT, GCTGCT, CAG, CAGCAG, AGC, AGCAGC, GCA, GCAGCA, CAGG, CAGGCAGG, AGGC, AGGCAGGC, GGCA, GGCAGGCA, GCAG, GCAGGCAG, AGAAT, GAATA, AATAG, ATAGA, TAGAA, GGCCCC, GCCCCG, CCCCGG, CCCGGC, CCGGCC, and CGGCCC. In another example of self-assembling genetic recognition reagents, International Patent Application Publication No. WO 2014/169216describes genetic recognition reagents that self-assemble and covalently-link in a reducing environment by virtue of terminal thioester and sulfhydryl groups. Covalent linkages form between unit self-assembling genetic recognition reagents when aligned contiguously on a target sequence, for example on a nucleic acid comprising an expanded repeat, e.g., as described above. Other end-groups, such as affinity binding partners, or reactive groups, can be attached to the ends of the genetic recognition reagents described herein to render them capable of self-assembly on a complementary nucleic acid template.

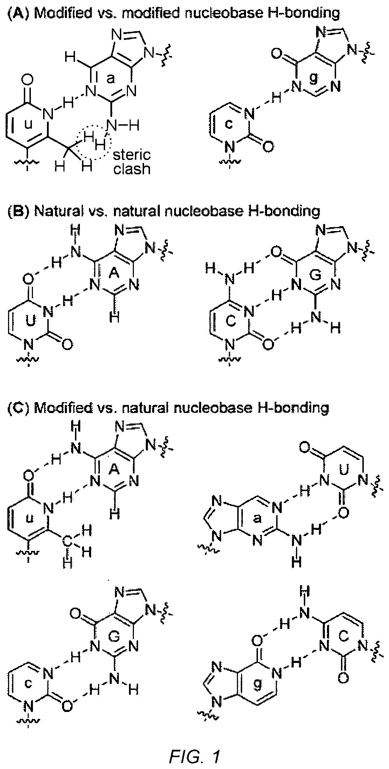

In one embodiment (not part of the claimed invention), provided herein is a compound comprising a nucleobase and a nucleic acid or nucleic acid analog backbone monomer. In the context of the present disclosure, a "nucleotide" refers to a compound or residue of a genetic recognition reagent comprising at least one nucleobase and a backbone element, which in a nucleic acid, such as RNA or DNA is ribose or deoxyribose. Nucleotide monomers also comprise reactive groups that permit polymerization under specific conditions. In native DNA and RNA, those reactive groups are the 5' phosphate and 3' hydroxyl groups. For chemical synthesis of nucleic acids and analogs thereof, the bases and backbone monomers may contain modified groups, such as blocked amines, as are known in the art. A "nucleotide residue" refers to a single nucleotide that is incorporated into an oligonucleotide or polynucleotide. Likewise, a "nucleobase residue" refers to a nucleobase incorporated into a nucleotide or a nucleic acid or analog thereof. A "genetic recognition reagent" refers generically to a nucleic acid or a nucleic acid analog that comprises a sequence of nucleobases that is able to hybridize to a complementary nucleic acid sequence on a nucleic acid by cooperative base pairing (e.g., Watson-Crick base pairing or Watson-Crick-like base pairing) (see, Figure 1). Intra-molecular base pairing does not occur between the modified bases described herein, where there is either steric clash or insufficient hydrogen bonding between bases that would normally base-pair in natural nucleic acid (Figure 1, panel (A) ("Figure 1 (A)")). As can be seen in Figure 1 (B), normal base-pairing is asymmetrical, with two hydrogen bonds forming between A and T/U nucleobases, and three hydrogen bonds forming between G and C nucleobases. Figure 1 (C) depicts a significant benefit of use of the modified nucleobases described herein, in that binding is symmetrical, with two hydrogen bonds forming between all combinations of the modified nucleobases described herein and their natural base-pairing partner. Not only does the near equal binding "weight" distribution of base-pairs greatly simplify the probe design, with the binding strength of the probe heavily reliance on length rather than on length and sequence composition, it confers greater sequence discrimination against base-pair mismatches than that could be achieved with natural nucleobases.

Figure 2 depicts a benefit of the modified nucleobases described herein. Figure 2 (A) shows that despite the (a'/a) sequence complementarity, a PNA oligomer (chiral or achiral) containing modified (u, c, a, and g) nucleobases cannot adopt a hairpin structure, but is able to hybridize to its complementary stem-loop RNA target. Figure 2 (B) shows that a tight-binding oligonucleotide molecule such as Locked Ncleic Acid (LNA) or PNA (e.g., yPNA) is able to invade the stem-loop structure, but its application in therapeutics and diagnostics poses considerable risks due to nonspecific binding. Figure 2 (C) shows that moderate avidity oligonucleotides, a category in which most oligonucleotide molecules fall under, are unable to open the stem-loop structure due to the lack of binding free energy, or as the result of the formation of a kinetic (hairpin) trap.

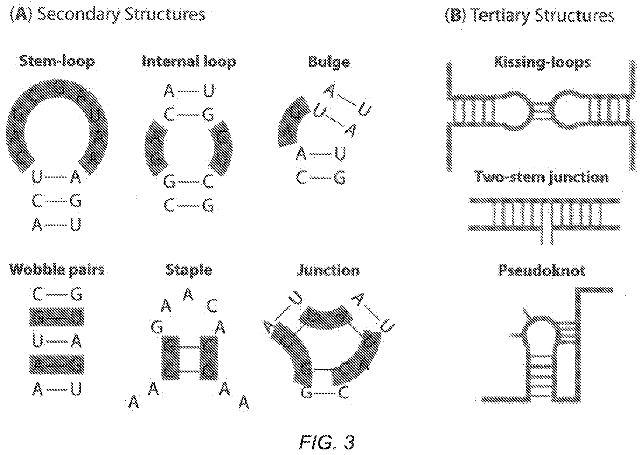

Examples of RNA secondary and tertiary structures that could be selectively targeted are shown in Figure 3. Potential therapeutic and diagnostic targets comprise RNA, both coding and noncoding, and DNA.

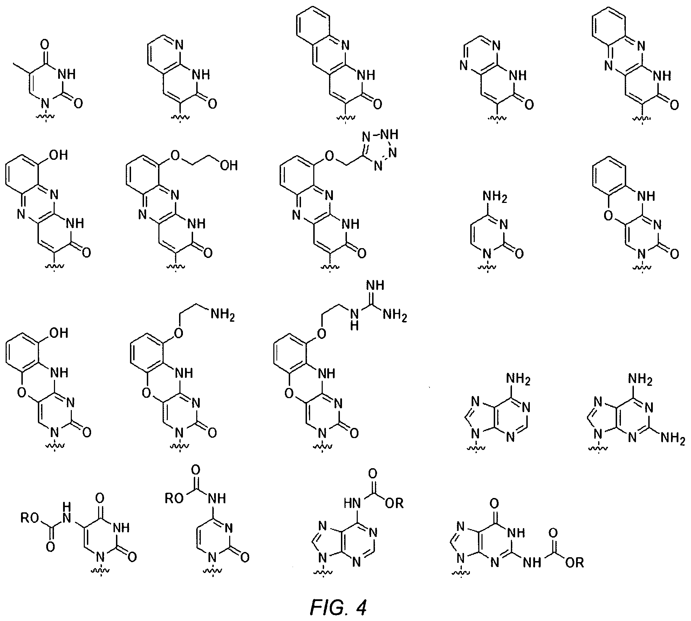

In aspects, provided herein are modified nucleobases. Nucleobases are recognition moieties that, e.g., bind specifically to one or more of adenine, guanine, thymine, cytosine, and uracil, e.g., by Watson-Crick or Watson-Crick-like base pairing by hydrogen bonding. A "nucleobase" includes primary (natural) nucleobases: adenine, guanine, thymine, cytosine, and uracil, as well as modified purine and pyrimidine bases, such as, without limitation, hypoxanthine, xanthene, 7-methylguanine, 5, 6, dihydrouracil, 5-methylcytosine, and 5-hydroxymethylcytosine. Figure 4 also depicts non-limiting examples of nucleobases, including monovalent nucleobases (e.g., adenine, cytosine, guanine, thymine or uracil, which bind to one strand of nucleic acid or nucleic acid analogs), and "clamp" nucleobases, such as a "G-clamp," which binds complementary nucleobases with enhanced strength. Additional purine, purine-like, pyrimidine and pyrimidine-like nucleobases are known in the art, for example as disclosed in United States Patent Nos. 8,053,212, 8,389,703, and 8,653,254. Divalent nucleobases are described in further detail in United States Patent Application Publication No. 2016/0083434 A1 and International Patent Application Publication No. WO/2018/058091 and bind two nucleobases instead of one, and therefore can form complex trimeric structures with matched or mismatched nucleic acids.

In one example (not part of the claimed invention) the backbone monomer is a ribose mono-, di-, or tri-phosphate or a deoxyribose mono-, di-, or tri-phosphate, such as a 5' monophosphate, diphosphate, or triphosphate of ribose or deoxyribose. The backbone monomer includes both the structural "residue" component, such as the ribose in RNA, and any active groups that are modified in linking monomers together, such as the 5' triphosphate and 3' hydroxyl groups of a ribonucleotide, which are modified when polymerized into RNA to leave a phosphodiester linkage. Likewise for PNA, the C-terminal carboxyl and N-terminal amine active groups of the N-(2-aminoethyl)glycine backbone monomer are condensed during polymerization to leave a peptide (amide) bond. In another aspect (not part of the claimed invention), the active groups are phosphoramidite groups useful for phosphoramidite oligomer synthesis, as is broadly-known in the arts. The nucleotide monomer also optionally comprises one or more protecting groups as are known in the art, such as 4,4'-dimethoxytrityl (DMT), and as described herein. A number of additional methods of preparing synthetic genetic recognition reagents are known, and depend on the backbone structure and particular chemistry of the base addition process. Determination of which active groups to utilize in joining nucleotide monomers and which groups to protect in the bases, and the required steps in preparation of oligomers is well within the abilities of those of ordinary skill in the chemical arts and in the particular field of nucleic acid and nucleic acid analog oligomer synthesis.

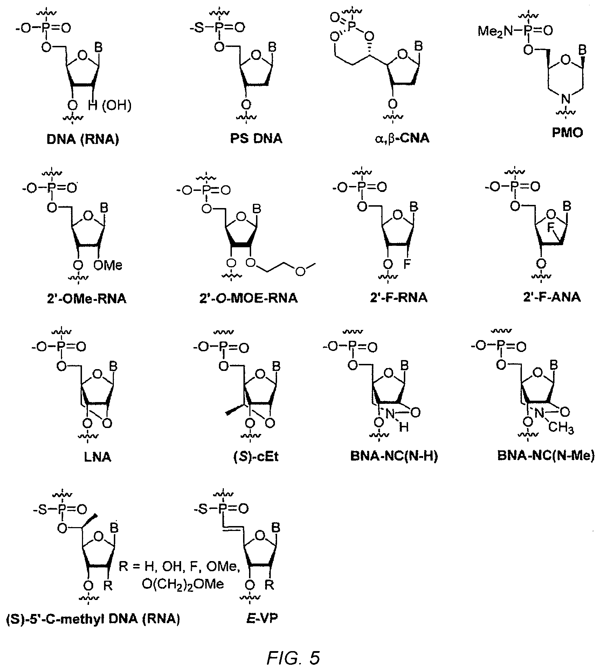

As used herein, the term "nucleic acid" refers to deoxyribonucleic acids (DNA) and ribonucleic acids (RNA). Nucleic acid analogs include, for example and without limitation (see, e.g., Figure 5): phosphorothioate DNA (PS DNA), α, β-constrained nucleic acid (α,β-CNA), 2'-methoxyl RNA, 2'-fluoro RNA, phosphorodiamidate morpholino oligomer (PMO), locked nucleic acid (LNA), 2',4'-constrained ethyl nucleic acid ((S)-cEt), 2',4' bridged nucleic acid NC (N-H) (BNA-NC(N-H)), 2',4' bridged nucleic acid NC (N-methyl) (BNA-NC(N-Me)), ((S)-5'-C-methyl DNA (RNA)), and 5'-E-vinylphosphonate nucleic acid (E-VP), wherein R is H, OH, F, OMe, or O(CH₂)₂OMe and combinations thereof including, optionally ribonucleotide or deoxyribonucleotide residue(s). An "oligonucleotide" is a short, single-stranded genetic recognition reagent. An oligonucleotide may be referred to by the length (i.e. number of nucleotides or nucleobases) of the strand, through the nomenclature "-mer". For example, an oligonucleotide of 22 nucleotides would be referred to as a 22-mer.

A "peptide nucleic acid" refers to a DNA or RNA analog or mimic in which the sugar phosphodiester backbone of the DNA or RNA is replaced by a N-(2-aminoethyl)glycine unit. In one embodiment, the peptide nucleic acid has the structure: 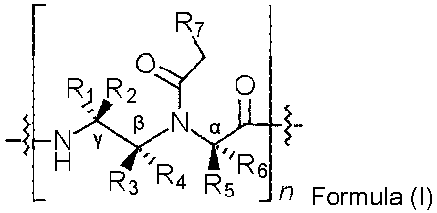 where n is 1 or greater, R₁, R₂, R₃, R₄, R₅, and R₆ are, independently: H; CH₃, CH₂OH, CH(CH₃)OH, CH₂SH, CH(CH₃)CH₃, CH₂CH(CH₃)CH₃, CH(CH₃)CH₂CH₃, CH₂CH₂SCH₃, CH₂CH₃, CH₂-C₆H₅, CH₂-C₆H₄OH, 1H-indol-3-yl methyl, CH₂C(O)OH, CH₂CH₂C(O)OH, CH₂C(O)NH₂, CH₂CH₂C(O)NH₂, 1*H*-imidazol-4-yl methyl, CH₂CH₂CH₂CH₂NH₂, or CH₂CH₂CH₂NHC(NH)NH₂; linear or branched (C₃-C₈)alkyl, (C₂-C₈)alkenyl, (C₂-C₈)alkynyl, (C₃-C₈)aryl, (C₃-C₈)cycloalkyl, (C₃-C₈)aryl(C₁-C₆)alkylene, (C₃-C₈)cycloalkyl(C₁-C₆)alkylene, a guanidine-containing group, CH₂-(OCH₂-CH₂)ₙ-OH, CH₂-(OCH₂-CH₂)ₙ-NH₂, CH₂-(OCH₂-CH₂)ₙ-SH, CH₂-(OCH₂-CH₂)ₙ-NHC(NH)NH₂, CH₂-(OCH₂-CH₂)ₙ-morpholine, CH₂-(OCH₂-CH₂)ₙ-piperazine, 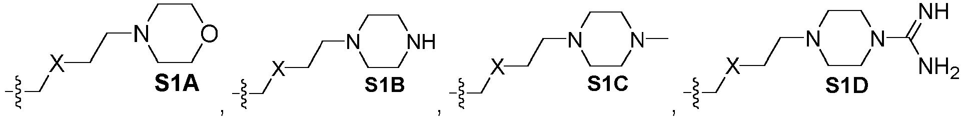 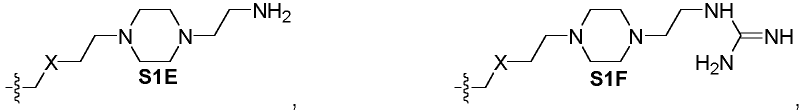 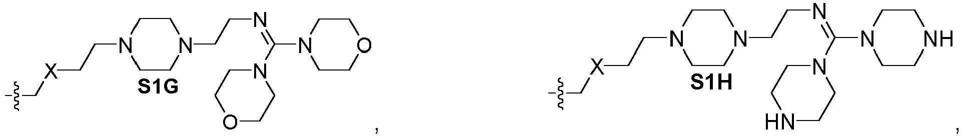 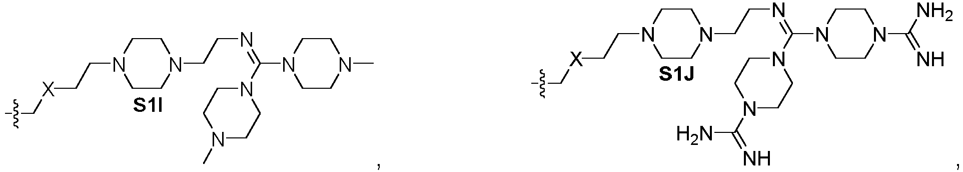 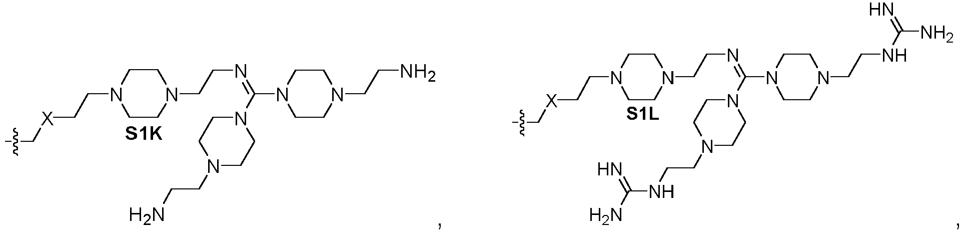 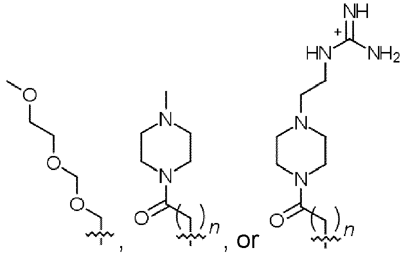 wherein X is a linker, such as a linear linker, R₁ and R₂ together form a 1,3-propylene linkage, R₃ and R₄ together form a 1,3-propylene linkage, or R₅ and R₆ together form a 1,3-propylene linkage, and each instance of R7 is, independently, a nucleobase, forming a nucleobase sequence where n is two or greater.

A linker is a moiety in a compound that covalently connects one moiety to another. It imparts no substantial negative effect on the activity of the overall compound, e.g., in context of the present invention, the ability of a genetic recognition reagent to operate in its intended use. Aside from serving to covalently-link two moieties, a linker may have a beneficial effect, such as in the physical separation of moieties to which it is attached, e.g., to optimize spacing to avoid steric effects. A linker also may serve some additional function, such as altering the hydrophobicity/hydrophilicity of the overall molecule, to provide an additional site (e.g., an amine protected by a protective group) for linking additional moieties to the compound, or to rigidize the overall molecule. A linker is attached to the remainder of the compound by any suitable linkage moiety ('linkage"), e.g., by a carbon-carbon bond, an ester, a thioester, an amine, an ether, an amide, a carbonate, or a carbamate linkage to the additional moieties of the compound. The linker may be hydrocarbyl, that is including only carbons and hydrogens (e.g. from 1 to 10 methylene groups), optionally comprising one or more hetero-atom, such as N, O, and/or S. In the context of the present invention, in one embodiment, one suitable linker is a divalent moiety comprising a PEG group -(O-CH₂-CH₂)ₙ-, where n ranges from 2 to 100, e.g., from 2-10 (PEG₂₋₁₀), or from 2-5 (PEG₂₋₅) such as 2 (PEG₂), 3, 4, 5, 6, 7, 8, 9, or 10. A PEG linker may comprise one or more methylene groups at either end in addition to a suitable linkages attaching the PEG group to connected moieties.

In another embodiment, the PNA is a gamma PNA (γPNA), which is an oligomer or polymer of gamma-modified N-(2-aminoethyl)glycine monomers where the γ carbon in formula (I), above, is a chiral center, typically with one of R₁ or R₂, attached to the gamma carbon being H and the other not a hydrogen, or R₁ and R₂ are different, such that the gamma carbon is a chiral center. When R₁ and R₂ are both hydrogen (N-(2-aminoethyl)-glycine backbone), or are the same, there is no such chirality about the gamma carbon. Alpha PNA (αPNA), is an oligomer or polymer of alpha-modified N-(2-aminoethyl)glycine monomers of the where the α carbon in formula (I), above, is a chiral center. Beta PNA (βPNA), is an oligomer or polymer of beta-modified N-(2-aminoethyl)glycine monomers of the where the β carbon in formula (I), above, is a chiral center. The discussion below regarding γPNA applies equally to αPNA and βPNA. Also, any combination of two (α,β, α,γ, or β,γ) or all three (α,β,γ) of the α, β, and γ carbons may form chiral centers.

In various embodiments (not part of the claimed invention), the backbone, e.g. at one or more of R₁, R₂, R₃, R₄, R₅, and R₆, is PEGylated with from 1 to 50 oxyethylene residues - that is, [-O-CH₂-CH₂-]ₙ, where n is 1 to 50, inclusive. The PEG group(s) can be linked to the backbone by any suitable linking group, such as by a C₁₋₆ alkyl group, or and aryl-alkyl group.

In other embodiments, the backbone, e.g., at one or more of R₁, R₂, R₃, R₄, R₅, and R₆, comprises one or more guanidine-containing groups, such as an alkyl or aryl-alkyl moiety terminated in a guanidine moiety.

In other embodiments, one or more of R₁, R₂, R₃, R₄, R₅, and R₆ are S1A, S1B, S1C, S1D, S1E, S1F, S1G, S1H, S1I, S1J, S1K,.or S1L, to promote cellular uptake and endosomal escape.

An "amino acid side chain" is a side chain for an amino acid. Amino acids have the structure: 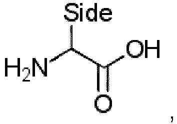 where "Side" is the amino acid side chain. Non-limiting examples of amino acid side chains include: CH₃ (Ala), CH₂OH (Ser), CH(CH₃)OH (Thr), CH₂SH (Cys), CH(CH₃)CH₃ (Val), CH₂CH(CH₃)CH₃ (Leu), CH(CH₃)CH₂CH₃ (Ile), CH₂CH₂SCH₃ (Met), 4-CH₂-C₆H₄OH (Tyr), CH₂-C₆H₅ (Phe), 1H-indol-3-yl methyl (Trp), CH₂C(O)OH (Asp), CH₂CH₂C(O)OH (Glu), CH₂C(O)NH₂ (Asn), CH₂CH₂C(O)NH₂ (Gln), 1H-imidazol-4-yl methyl (His), CH₂CH₂CH₂CH₂NH₂ (Lys), or CH₂CH₂CH₂NHC(NH)NH₂ (Arg, comprising a guanidine/guanidium group). Glycine is not represented because in the embodiment there is no side chain (Side is H).

A γPNA monomer incorporated into a γPNA oligomer or polymer is referred to herein as a "γPNA monomer residue", with each residue having the same or different nucleobase, such as the modified nucleobases described herein, such that the order of bases on the γPNA is its "sequence", as with DNA or RNA. A sequence of nucleobases in a nucleic acid or a nucleic acid analog oligomer or polymer, such as a γPNA oligomer or polymer, binds to a complementary sequence of adenine, guanine, cytosine, thymine and/or uracil residues in a nucleic acid strand by cooperative bonding, essentially as with Watson-Crick binding of complementary bases in double-stranded DNA or RNA. "Watson-Crick-like" bonding refers to hydrogen bonding of nucleobases other than G, A, T, C or U, such as the bonding of the divalent bases shown herein with G, A, T, C, U or other nucleobases.

Unless otherwise indicated, the nucleic acids and nucleic acid analogs described herein are not described with respect to any particular sequence of bases. The present disclosure is directed to modified nucleobases, compositions comprising the modified nucleobases, and methods of use of the modified nucleobases and compounds containing those nucleobases, and the usefulness of any specific embodiments described herein, while typically depending upon a specific sequence in each instance, is generically applicable. A nucleobase sequence attached to the backbone of γPNA oligomers can hybridize with a complementary nucleobase sequence of a target nucleic acid or nucleic acid analog by Watson-Crick or Watson-Crick-like hydrogen bonding. One of ordinary skill would understand that the compositions and methods described herein are sequence-independent and describe novel, generalized compositions comprising divalent nucleobases and related methods.

Genetic recognition reagents can be prepared as small oligonucleotides and can be assembled *in situ, in vivo, ex vivo,* or *in vitro,* for example, as described in United States Patent Application Publication No. 2016/0083433 A1. By that method, small oligomers of high cell or tissue permeability as compared to longer sequences, such as trimers, can be transferred to a cell, and the oligomers can be assembled as a contiguous larger sequence once hybridized to a template nucleic acid. The same can be accomplished *in vitro* or ex *vivo,* for example, for rapidly assembling a longer sequence for use in hybridizing to a target nucleic acid.

In one aspect, the genetic recognition reagent is provided on an array (not part of the claimed invention). Arrays are particularly useful in implementing high-throughput assays, such as genetic detection assays. As used herein, the term "array" refers to reagents, for example the genetic recognition reagents described herein, located or attached at two or more discrete, identifiable and/or addressable locations on a substrate. In one aspect, an array is an apparatus having two or more discrete, identifiable reaction chambers, such as, without limitation a 96-well dish, in which reactions comprising identified constituents are performed. In one aspect, two or more genetic recognition reagents comprising one or more divalent nucleobases as described herein are immobilized onto a substrate in a spatially addressable manner so that each individual primer or probe is located at a different and (addressable) identifiable location on the substrate. One or more genetic recognition reagent is either covalently-linked to the substrate or are otherwise bound or located at addressable locations on the array. Substrates include, without limitation, multi-well plates, silicon chips and beads. In one aspect, the array comprises two or more sets of beads, with each bead set having an identifiable marker, such as a quantum dot or fluorescent tag, so that the beads are individually identifiable using, for example and without limitation, a flow cytometer. In one aspect, an array is a multi-well plate containing two or more wells with the described genetic recognition reagents for binding specific sequences. As such, reagents, such as probes and primers may be bound or otherwise deposited onto or into, or otherwise located at specific locations on an array. Reagents may be in any suitable form, including, without limitation: in solution, dried, lyophilized, or glassified. When linked covalently to a substrate, such as an agarose bead or silicon chip, a variety of linking technologies are known for attaching chemical moieties, such as the genetic recognition reagents to such substrates.

Linkers and spacers for use in linking nucleic acids, peptide nucleic acids and other nucleic acid analogs are broadly known in the chemical and array arts and for that reason are not described herein. As a non-limiting example, a γPNA genetic recognition reagent contains a reactive amine, which can be reacted with carboxyl-, cyanogen bromide-, N-hydroxysuccinimide ester-, carbonyldiimidazole-, or aldehyde-functional agarose beads, available, for instance from Thermo Fisher Scientific (Pierce Protein Biology Products), Rockford, Illinois, and a variety of other sources. The genetic recognition reagents described herein can be attached to a substrate in any manner, with or without linkers. Devices for use in conducting reactions, and for reading arrays are broadly-known and available, and informatics and/or statistical software or other computer-implemented processes for analyzing array data and/or identifying genetic risk factors from data obtained from a patient sample, are known in the art.

Certain of the modified nucleobases described herein exhibit fluorescence, due to their ring structure. These compositions can be used as fluorochromes, or the intrinsic fluorescence can be employed as a probe, for example, by binding target sequences in an *in situ* assay or in a gel or blot, such that a target sequence can be visualized.

According to one aspect of the present invention (not part of the claimed invention), a method is provided for detection of a target sequence in a nucleic acid, comprising contacting a genetic recognition reagent composition as described herein with a sample comprising nucleic acid and detecting binding of the genetic recognition reagent with a nucleic acid. In one aspect (not part of the claimed invention), the genetic recognition reagent is immobilized on a substrate, for example in an array, and labeled (e.g., fluorescently labeled or radiolabeled) nucleic acid sample is contacted with the immobilized genetic recognition reagent and the amount of labeled nucleic acid specifically bound to the genetic recognition reagent is measured. In a variation (not part of the claimed invention), genetic recognition reagent or a nucleic acid comprising a target sequence of the genetic recognition reagent is bound to a substrate, and a labeled nucleic acid comprising a target sequence of the genetic recognition reagent or a labeled genetic recognition reagent is bound to the immobilized genetic recognition reagent or nucleic acid, respectively to form a complex. In one aspect (not part of the claimed invention), the nucleic acid of the complex comprises a partial target sequence so that a nucleic acid comprising the full target sequence would out-compete the complexed nucleic acid for the genetic recognition reagent. The complex is then exposed to a nucleic acid sample and loss of bound label from the complex could be detected and quantified according to standard methods, facilitating quantification of a nucleic acid marker in the nucleic acid sample. These are merely two of a large number of possible analytical assays that can be used to detect or quantify the presence of a specific nucleic acid in a nucleic acid sample.

By "immobilized" in reference to a composition such as a nucleic acid or genetic recognition reagent as described herein, it is meant attached to a substrate of any physical structure or chemical composition. The immobilized composition is immobilized by any method useful in the context of the end use. The composition is immobilized by covalent or non-covalent methods, such as by covalent linkage of amine groups to a linker or spacer, or by non-covalent bonding, including Van der Waals and/or hydrogen bonding. A "label" is a chemical moiety that is useful in detection of, or purification or a molecule or composition comprising the label. A label may be, for example and without limitation, a radioactive moiety, such as ¹⁴C, ³²P, ³⁵S, a fluorescent dye, such as fluorescein isothiocyanate or a cyanine dye, an enzyme, or a ligand for binding other compounds such as biotin for binding streptavidin, or an epitope for binding an antibody. A multitude of such labels, and methods of use thereof are known to those of ordinary skill in the immunology and molecular biology arts. That said, because certain nucleobases described herein are fluorescent, incorporation of such bases into nucleotide residues of a nucleic acid or nucleic acid analog, or covalently-linking a divalent nucleobase to a nucleic acid, nucleic acid analog, binding reagent, ligand or other detection reagent can permit detection of and/or quantification of a reagent in a sample, reaction mixture, array, etc.

In yet another aspect of the present invention (not part of the claimed invention), a method of isolation and purification or a nucleic acid containing a target sequence is provided. In one non-limiting aspect (not part of the claimed invention), a genetic recognition reagent as described herein is immobilized on a substrate, such as a bead (for example and without limitation, an agarose bead, a bead containing a fluorescent marker for sorting, or a magnetic bead), porous matrix, surface, tube, *etc.* A nucleic acid sample is contacted with the immobilized genetic recognition reagent and nucleic acids containing the target sequence bind to the genetic recognition reagent. The bound nucleic acid is then washed to remove unbound nucleic acids, and the bound nucleic acid is then eluted, and can be precipitated or otherwise concentrated by any useful method as are broadly known in the molecular biological arts.

In a further aspect (not part of the claimed invention), kits are provided. A kit comprises at a minimum a vessel of any form, including cartridges for automated nucleic acid, nucleic acid analog, or PNA synthesis, which may comprise one or more vessels in the form of individual and independent, optionally independently-addressable compartments, for use, for example, in an automatic sequence preparation device for preparing nucleic acids and/or nucleic acid analogs. Vessels may be single-use, or contain sufficient contents for multiple uses. A kit also may comprise an array. A kit may optionally comprise one or more additional reagents for use in making or using genetic recognition reagents in any embodiment described herein. The kit comprises a vessel containing any divalent nucleobase in any form described herein, or monomers or genetic recognition reagents according to any aspect described herein. Different nucleobases, monomers or genetic recognition reagents are typically packaged into separate vessels, which may be separate compartments in a cartridge.

In aspects (not part of the claimed invention), the compounds and genetic recognition reagents are used for therapeutic purposes and therefore those compounds and genetic recognition reagents are formulated in a drug product, pharmaceutical composition, or dosage form, including compositions for human and veterinary use, including a therapeutically effective amount of the compound or genetic recognition reagent and an excipient, e.g., a vehicle or diluent for therapeutic delivery, e.g., and without limitation, for oral, topical, intravenous, intramuscular, or subcutaneous administration. The composition can be formulated in a classical manner using solid or liquid vehicles, diluents and additives appropriate to the desired mode of administration. Orally, the compounds can be administered in the form of tablets, capsules, granules, powders and the like. The compositions optionally comprise one or more additional active agents, as are broadly known in the pharmaceutical, medicinal, veterinary or biological arts. The compounds described herein may be administered in any effective manner. Further examples of delivery routes include, without limitation: topical, for example, epicutaneous, inhalational, enema, ocular, otic and intranasal delivery; enteral, for example, orally, by gastric feeding tube or swallowing, and rectally; and parenteral, such as, intravenous, intraarterial, intramuscular, intracardiac, subcutaneous, intraosseous, intradermal, intrathecal, intraperitoneal, transdermal, iontophoretic, transmucosal, epidural and intravitreal. Therapeutic/pharmaceutical compositions are prepared in accordance with acceptable pharmaceutical procedures, as are broadly-known.

Any of the compounds described herein may be compounded or otherwise manufactured into a suitable composition for use, such as a pharmaceutical dosage form or drug product in which the compound or genetic recognition reagent is an active ingredient (not part of the claimed invention). According to one example (not part of the claimed invention), the drug product described herein is an oral tablet, capsule, caplet, liquid-filled or gel-filled capsule, *etc.* Compositions may comprise a pharmaceutically acceptable carrier, or excipient. An "excipient" is an inactive substance used as a carrier for the active ingredients of a medication. Although "inactive," excipients may facilitate and aid in increasing the delivery, stability or bioavailability of an active ingredient in a drug product. Non-limiting examples of useful excipients include: antiadherents, binders, rheology modifiers, coatings, disintegrants, emulsifiers, oils, buffers, salts, acids, bases, fillers, diluents, solvents, flavors, colorants, glidants, lubricants, preservatives, antioxidants, sorbents, vitamins, sweeteners, etc., as are available in the pharmaceutical/compounding arts.

### Example - Synthesis of modified nucleobases and cell-permeable γPNAs

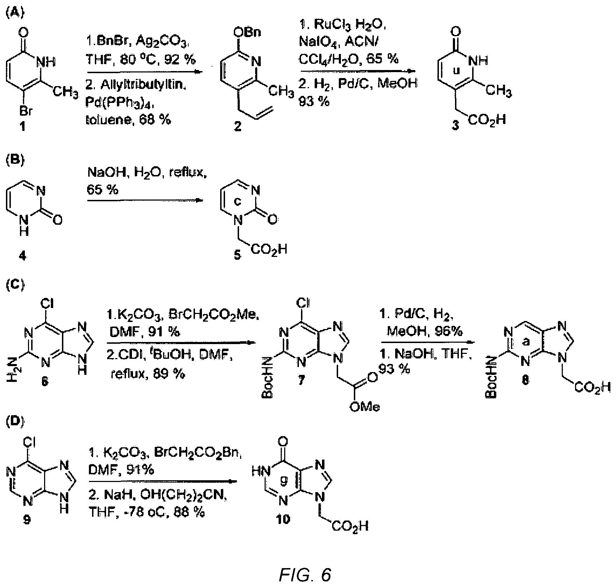

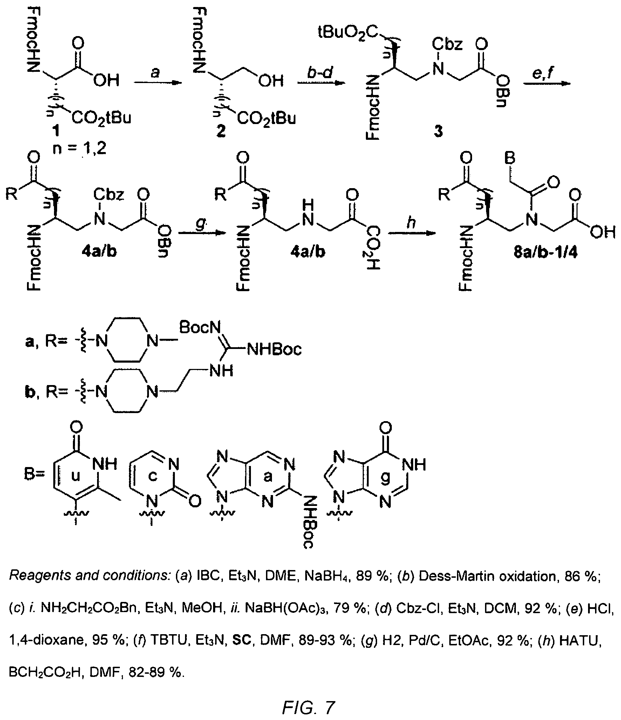

The compounds and genetic recognition reagents described herein are synthesized according to methods known in the chemical and organic synthesis arts. Illustrative synthesis schemes are provided in Figures 6 and 7.

## Claims

1. A genetic recognition reagent comprising a plurality of nucleobase moieties attached to a nucleic acid or nucleic acid analog backbone, in which at least one nucleobase moiety is: 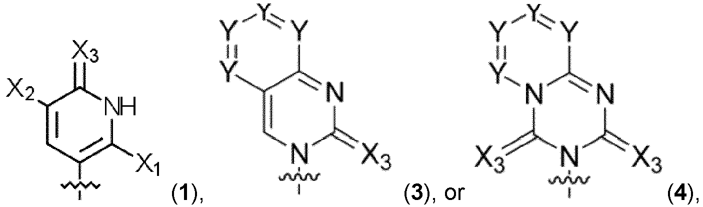 wherein,
X₁ is CH₃;
X₂ is H, CH₃, CN, NC, N₃, C(O)OH, or C(O)NH₂;
X₃ is O or S;
and
Y is N or CH.

2. The genetic recognition reagent of claim 1, wherein the at least one nucleobase moiety is:

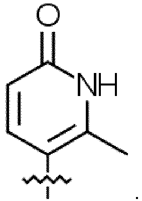

3. The genetic recognition reagent of claims 1 or 2, in which the backbone is chosen from one of a DNA, RNA, peptide nucleic acid (PNA), phosphorothioate DNA (PS DNA), α, β-constrained nucleic acid (α,β-CNA), 2'-methoxyl RNA, 2'-fluoro RNA, locked nucleic acid (LNA), 2',4'-constrained ethyl nucleic acid ((S)-cEt), 2',4'-bridged nucleic acid NC (N-H) (BNA-NC(N-H)), 2',4' bridged nucleic acid NC (N-methyl) (BNA-NC(N-Me)), 2'-(R)-(S)-5'-C-methyl DNA, or 2'-R-5'-E-vinylphosphonate nucleic acid (E-VP), wherein R is H, OH, F, OMe, or O(CH₂)₂OMe backbone.

4. The genetic recognition reagent of claim 1, in which the backbone is a peptide nucleic acid (PNA) backbone.

5. The genetic recognition reagent of claim 4, wherein the backbone comprises one or more guanidine moieties linked to the backbone.

6. The genetic recognition reagent of claim 1, in which the backbone is a gamma peptide nucleic acid (yPNA) backbone.

7. The genetic recognition reagent of claim 1, in which the backbone is a PNA backbone comprising the residue 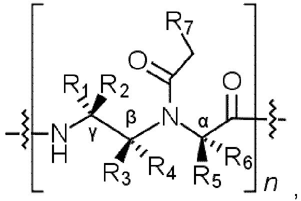 where n is 1 or greater, R₁, R₂, R₃, R₄, R₅, and R₆ are, independently: H; CH₃, CH₂OH, CH(CH₃)OH, CH₂SH, CH(CH₃)CH₃, CH₂CH(CH₃)CH₃, CH(CH₃)CH₂CH₃, CH₂CH₂SCH₃, CH₂CH₃, CH₂-C₆H₅, CH₂-C₆H₄OH, 1H-indol-3-yl methyl, CH₂C(O)OH, CH₂CH₂C(O)OH, CH₂C(O)NH₂, CH₂CH₂C(O)NH₂, 1H-imidazol-4-yl methyl, CH₂CH₂CH₂CH₂NH₂, or CH₂CH₂CH₂NHC(NH)NH₂; linear or branched (C₃-C₈)alkyl, (C₂-C₈)alkenyl, (C₂-C₈)alkynyl, (C₃-C₈)aryl, (C₃-C₈)cycloalkyl, (C₃-C₈)aryl(C₁-C₆)alkylene, (C₃-C₈)cycloalkyl(C₁-C₆)alkylene, a guanidine-containing group, CH₂-(OCH₂-CH₂)ₙ-OH, CH₂-(OCH₂-CH₂)ₙ-NH₂, CH₂-(OCH₂-CH₂)ₙ-SH, CH₂-(OCH₂-CH₂)ₙ-NHC(NH)NH₂, CH₂-(OCH₂-CH₂)ₙ-morpholine, CH₂-(OCH₂-CH₂)ₙ-piperazine, 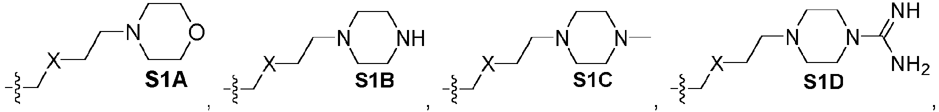 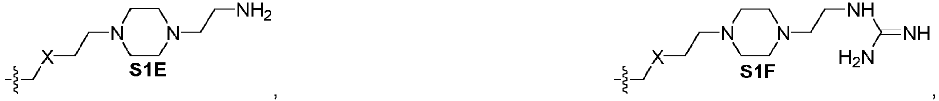 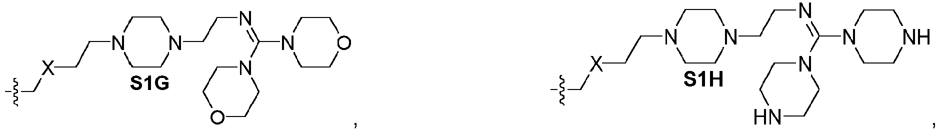 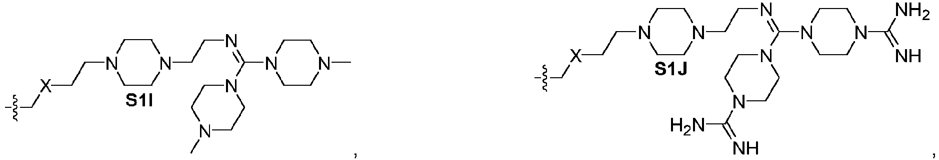 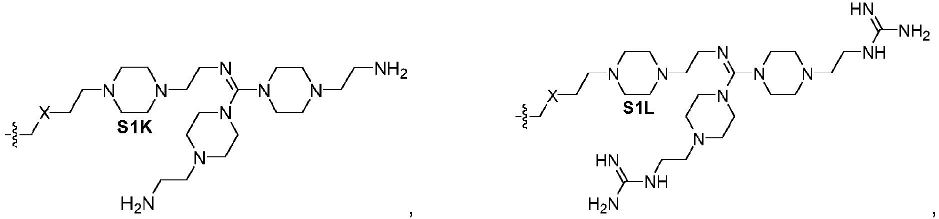 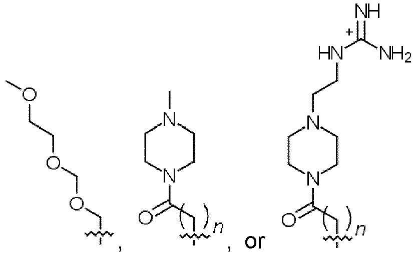 wherein X is a linker, R₁ and R₂ together form a 1,3-propylene linkage, R₃ and R₄ together form a 1,3-propylene linkage, or R₅ and R₆ together form a 1,3-propylene linkage, and each instance of R₇ is, independently, a nucleobase.

8. The genetic recognition reagent of claim 7, wherein at least one of R₁, R₂, R₃, R₄, R₅, and R₆ is S1A, S1B, S1C, S1D, S1E, S1F, S1G, S1H, S1I, S1J, S1K, or S1L.

9. The genetic recognition reagent of claim 7 or 8, wherein the α-carbon, the β-carbon, or the γ-carbon is a chiral center.

10. The genetic recognition reagent of claim 9, wherein the γ-carbon is a chiral center.

11. The genetic recognition reagent of claim 10, wherein R₂ is:

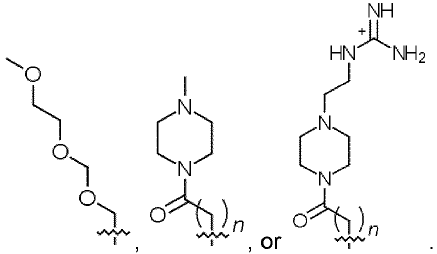

12. The genetic recognition reagent of claim 9, wherein R₁, R₃, R₄, R₅, and R₆ are H.

13. The genetic recognition reagent of any one of claims 1-12, wherein the plurality of nucleobase moieties form a sequence that comprises: TTC, TTCTTC, TCT, TCTTCT, CTT, CTTCTT, CCG, CCGCCG, CGC, CGCCGC, GCC, GCCGCC, CGG, CGGCGG, GCG, GCGGCG, GGC, GGCGGC, CTG, CTGCTG, TGC, TGCTGC, GCT, GCTGCT, CAG, CAGCAG, AGC, AGCAGC, GCA, GCAGCA, CAGG, CAGGCAGG, AGGC, AGGCAGGC, GGCA, GGCAGGCA, GCAG, GCAGGCAG, AGAAT, GAATA, AATAG, ATAGA, TAGAA, GGCCCC, GCCCCG, CCCCGG, CCCGGC, CCGGCC, and CGGCCC, or a contiguous repeat of any of the preceding.

14. The genetic recognition reagent of any one of claims 1-13, in which the plurality of nucleobase moieties is arranged in a sequence complementary to a target sequence of a nucleic acid.

15. The genetic recognition reagent of any one of claims 1-14, having from 3 to 25 nucleobase moieties.

## Patentansprüche

1. Genetisches Erkennungsreagenz, umfassend eine Vielzahl von Nukleobaseneinheiten, die an ein Nukleinsäure- oder ein Nukleinsäureanalogon-Grundgerüst gebunden sind, in welchem mindestens eine Nukleobaseneinheit 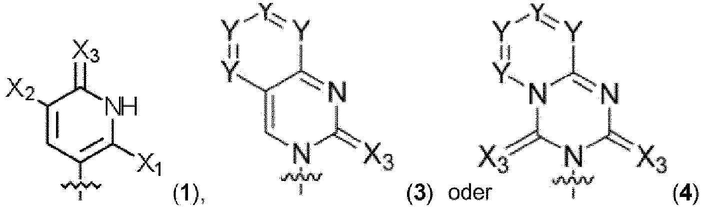 ist, wobei
X₁ CH₃ ist;
X₂ H, CH₃, CN, NC, N₃, C(O)OH oder C(O)NH₂ ist;
X₃ O oder S ist
und
Y N oder CH ist.

2. Genetisches Erkennungsreagenz nach Anspruch 1, wobei die mindestens eine Nukleobasen-Einheit 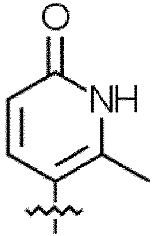 ist.

3. Genetisches Erkennungsreagenz nach Anspruch 1 oder 2, in welchem das Grundgerüst ausgewählt ist aus einem von DNA, RNA, Peptidnukleinsäure (PNA), Phosphorothioat-DNA (PS-DNA), α, β-eingeschränkter Nukleinsäure (α,β-CNA), 2'-Methoxyl-RNA, 2'-Fluor-RNA, gesperrter Nukleinsäure (LNA), 2',4'-eingeschränkter Ethylnukleinsäure ((S)-cEt), 2',4'-verbrückter Nukleinsäure NC (N-H) (BNA-NC(N-H)), 2',4'-verbrückter Nukleinsäure NC (N-Methyl) (BNA-NC(N-Me)), 2'-(R)-(S)-5'-C-methyl-DNA oder 2'-R-5'-E-vinylphosphonat-Nukleinsäure (E-VP), wobei R H, OH, F, OMe oder ein O(CH₂)₂OMe-Grundgerüst ist.

4. Genetisches Erkennungsreagenz nach Anspruch 1, in welchem das Grundgerüst ein Peptidnukleinsäure (PNA)-Grundgerüst ist.

5. Genetisches Erkennungsreagenz nach Anspruch 4, wobei das Grundgerüst eine oder mehrere Guanidin-Einheiten, die an das Grundgerüst gebunden sind, umfasst.

6. Genetisches Erkennungsreagenz nach Anspruch 1, in welchem das Grundgerüst ein Gamma-Peptidnukleinsäure (γPNA)-Grundgerüst ist.

7. Genetisches Erkennungsreagenz nach Anspruch 1, in welchem das Grundgerüst ein PNA-Grundgerüst ist, welches den Rest 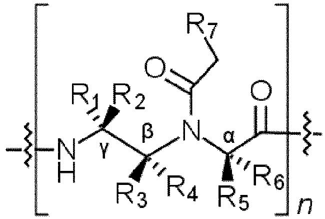 umfasst, wobei n 1 oder größer ist, R₁, R₂, R₃, R₄, R₅ und R₆ unabhängig voneinander sind: H; CH₃, CH₂OH, CH(CH₃)OH, CH₂SH, CH(CH₃)CH₃, CH₂CH(CH₃)CH₃, CH(CH₃)CH₂CH₃, CH₂CH₂SCH₃, CH₂CH₃, CH₂-C₆H₅, CH₂-C₆H₄OH, 1*H*-Indol-3-ylmethyl, CH₂C(O)OH, CH₂CH₂C(O)OH, CH₂C(O)NH₂, CH₂CH₂C(O)NH₂, 1H-Imidazol-4-ylmethyl, CH₂CH₂CH₂CH₂NH₂ oder CH₂CH₂CH₂NHC(NH)NH₂, lineares oder verzweigtes (C₃-C₈)Alkyl, (C₂-C₈)Alkenyl, (C₂-C₈)Alkinyl, (C₃-C₈)Aryl, (C₃-C₈)Cycloalkyl, (C₃-C₈)Aryl(C₁-C₆)alkylen, (C₃-C₈)Cycloalkyl(C₁-C₆)alkylen, eine Guanidin enthaltende Gruppe, CH₂-(OCH₂-CH₂)ₙ-OH, CH₂-(OCH₂-CH₂)ₙ-NH₂, CH₂-(OCH₂-CH₂)ₙ-SH, CH₂-(OCH₂-CH₂)ₙ-NHC(NH)NH₂, CH₂-(OCH₂-CH₂)ₙ-morpholin, CH₂-(OCH₂-CH₂)ₙ-piperazin, 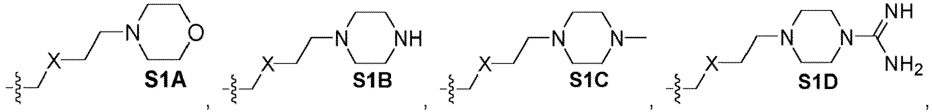 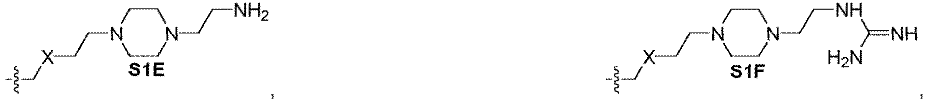 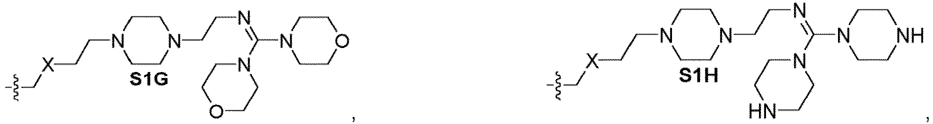 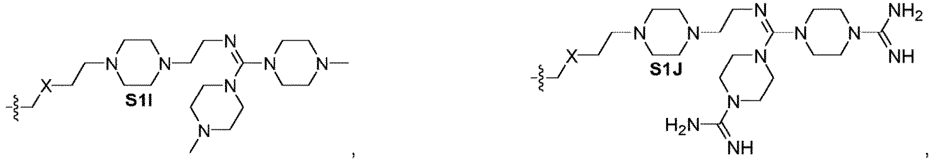 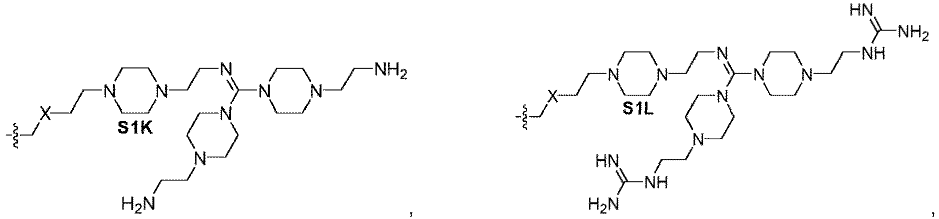 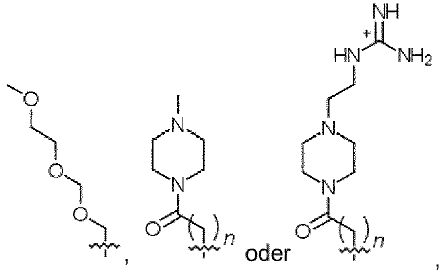 wobei X ein Linker ist, R₁ und R₂ zusammen eine 1,3-Propylenverbindung bilden, R₃ und R₄ zusammen eine 1,3-Propylenverbindung bilden oder R₅ und R₆ zusammen eine 1,3-Propylenverbindung bilden und jeder Fall von R₇ unabhängig eine Nukleobase ist.

8. Genetisches Erkennungsreagenz nach Anspruch 7, wobei mindestens einer der Reste R₁, R₂, R₃, R₄, R₅ und R₆ S1A, S1B, S1C, S1D, S1E, S1F, S1G, S1H, S1I, S1J, S1K oder S1L ist.

9. Genetisches Erkennungsreagenz nach Anspruch 7 oder 8, wobei der α-Kohlenstoff, der β-Kohlenstoff oder der γ-Kohlenstoff ein chirales Zentrum ist.

10. Genetisches Erkennungsreagenz nach Anspruch 9, wobei der γ-Kohlenstoff ein chirales Zentrum ist.

11. Genetisches Erkennungsreagenz nach Anspruch 10, wobei R₂ 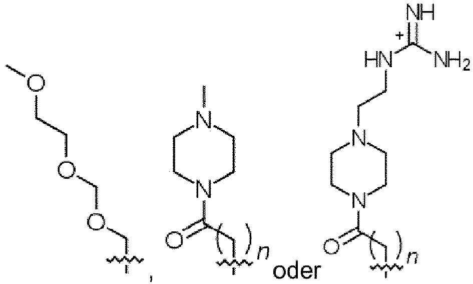 ist.

12. Genetisches Erkennungsreagenz nach Anspruch 9, wobei R₁, R₃, R₄, R₅ und R₆ H sind.

13. Genetisches Erkennungsreagenz nach einem der Ansprüche 1 bis 12, wobei die Vielzahl von Nukleobaseneinheiten eine Sequenz bilden, welche umfasst: TTC, TTCTTC, TCT, TCTTCT, CTT, CTTCTT, CCG, CCGCCG, CGC, CGCCGC, GCC, GCCGCC, CGG, CGGCGG, GCG, GCGGCG, GGC, GGCGGC, CTG, CTGCTG, TGC, TGCTGC, GCT, GCTGCT, CAG, CAGCAG, AGC, AGCAGC, GCA, GCAGCA, CAGG, CAGGCAGG, AGGC, AGGCAGGC, GGCA, GGCAGGCA, GCAG, GCAGGCAG, AGAAT, GAATA, AATAG, ATAGA, TAGAA, GGCCCC, GCCCCG, CCCCGG, CCCGGC, CCGGCC und CGGCCC oder eine zusammenhängende Wiederholung irgendeiner der vorstehenden Sequenzen.

14. Genetisches Erkennungsreagenz nach einem der Ansprüche 1 bis 13, wobei die Vielzahl von Nukleobaseneinheiten in einer zu einer Zielsequenz einer Nukleinsäure komplementären Sequenz angeordnet ist.

15. Genetisches Erkennungsreagenz nach einem der Ansprüche 1 bis 14, mit 3 bis 25 Nukleobaseneinheiten.

## Revendications

1. Réactif de reconnaissance génétique comprenant une pluralité de groupements de bases azotées attachés à un squelette d'acide nucléique ou d'analogue d'acide nucléique, dans lequel au moins un groupement de bases azotées est : 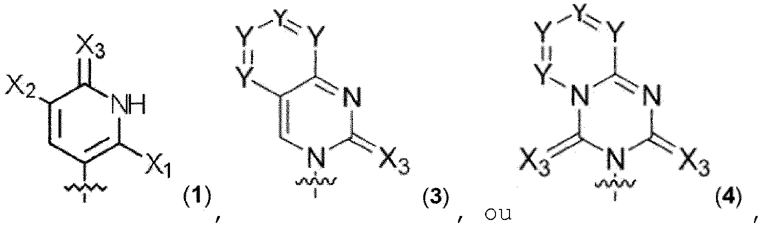 dans lequel
X₁ est CH₃ ;
X₂ est H, CH₃, CN, NC, N₃, C(O)OH ou C(O)NH₂ ;
X₃ est O ou S ; et
Y est N ou CH.

2. Réactif de reconnaissance génétique selon la revendication 1, dans lequel ledit au moins un groupement de bases azotées est :

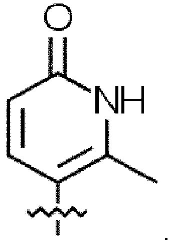

3. Réactif de reconnaissance génétique selon la revendication 1 ou 2, dans lequel le squelette est choisi parmi un ADN, un ARN, un acide nucléique peptidique (ANP), un ADN thiophosphate (PS DNA), un acide nucléique α,β-contraint (α,β-CNA), un ARN 2'-méthoxyle, un ARN 2'-fluoré, un acide nucléique bloqué (LNA), un acide nucléique éthylique 2',4'-contraint ((S)-cEt), un acide nucléique ponté en 2',4' NC(NH) (BNA-NC(NH)), un acide nucléique ponté en 2',4' NC(N-méthyle) (BNA-NC(N-Me)), un ADN 2'-(R)-(S)-5'-C-méthyle, ou un acide nucléique 2'-R-5'-E-vinylphosphonate (E-VP), dans lequel R est H, OH, F, OMe, ou un squelette O(CH₂)₂OMe.

4. Réactif de reconnaissance génétique selon la revendication 1, dans lequel le squelette est un squelette d'acide nucléique peptidique (ANP).

5. Réactif de reconnaissance génétique selon la revendication 4, dans lequel le squelette comprend une ou plusieurs groupements guanidine liées au squelette.

6. Réactif de reconnaissance génétique selon la revendication 1, dans lequel le squelette est un squelette acide nucléique peptidique gamma (γANP).

7. Réactif de reconnaissance génétique selon la revendication 1, dans lequel le squelette est un squelette ANP comprenant le résidu 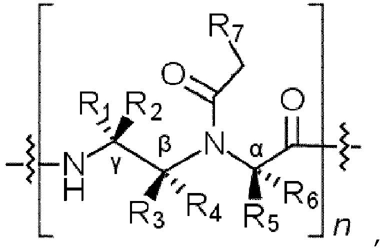 où n est supérieur ou égal à 1, R₁, R₂, R₃, R₄, R₅ et R₆ sont, indépendamment : H ; CH₃, CH₂OH, CH(CH₃) OH, CH₂SH, CH(CH₃)CH₃, CH₂CH(CH₃)CH₃, CH(CH₃)CH₂CH₃, CH₂CH₂SCH₃, CH₂CH₃, CH₂-C₆H₅, CH₂-C₆H₄OH, 1H-indol-3-yl méthyle, CH₂C(O)OH, CH₂CH₂C(O)OH, CH₂C(O)NH₂, CH₂CH₂C(O)NH₂, 1H-imidazol-4-yl méthyle, CH₂CH₂CH₂CH₂NH₂, ou CH₂CH₂ CH₂NHC(NH)NH₂ ; un (C₃-C₈)alkyle linéaire ou ramifié, un (C₂-C₈)alcényle, un (C₂-C₈)alcynyle, un (C₃-C₈)aryle, un (C₃-C₈)cycloalkyle, un (C₃-C₈)aryl(C₁-C₆)alkylène, un (C₃-C₈)cycloalkyl(C₁-C₆)alkylène, un groupe contenant une guanidine, CH₂-(OCH₂-CH₂)ₙ-OH, CH₂-(OCH₂-CH₂)ₙ-NH₂, CH₂-(OCH₂-CH₂)ₙ-SH, CH₂-(OCH₂-CH₂)ₙ-NHC (NH)NH₂, CH₂-(OCH₂-CH₂)ₙ-morpholine, CH₂-(OCH₂-CH₂)ₙ-pipérazine, 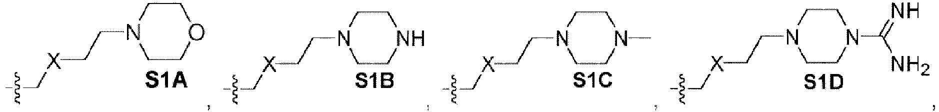 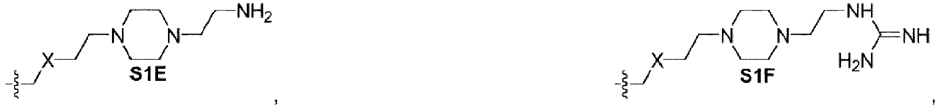 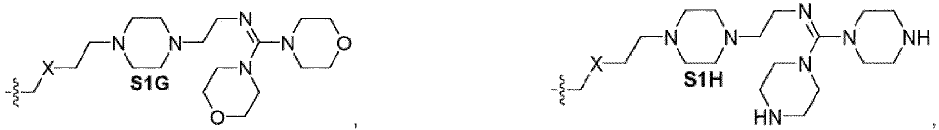 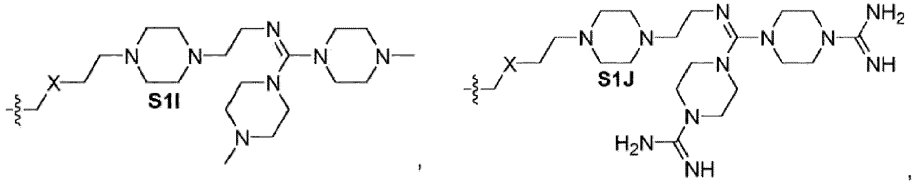 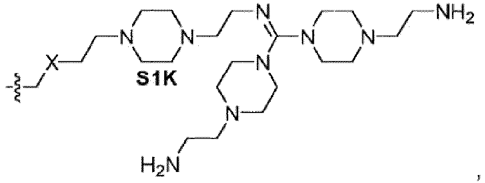 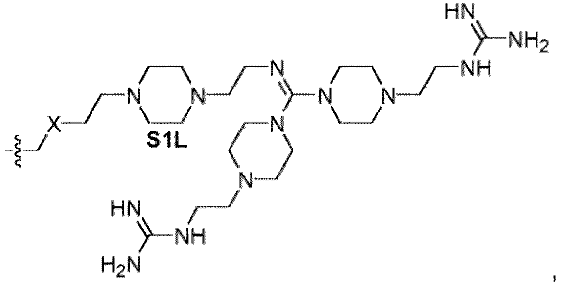 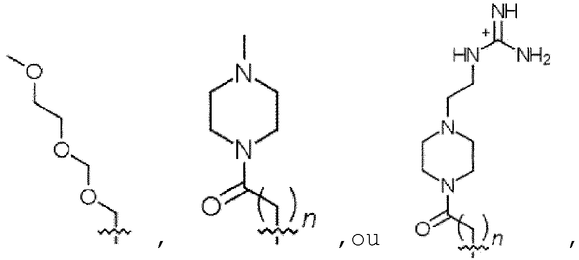 dans lequel X est un groupe de liaison, R₁ et R₂ forment ensemble une liaison 1,3-propylène, R₃ et R₄ forment ensemble une liaison 1,3-propylène, ou R₅ et R₆ forment ensemble une liaison 1,3-propylène, et chaque instance de R₇ est, indépendamment, une base azotée.

8. Réactif de reconnaissance génétique selon la revendication 7, dans lequel au moins l'un des groupes R₁, R₂, R₃, R₄, R₅ et R₆ est S1A, S1B, S1C, S1D, S1E, S1F, S1G, S1H, S1I, S1J, S1K ou S1L.

9. Réactif de reconnaissance génétique selon la revendication 7 ou 8, dans lequel le carbone α, le carbone β ou le carbone y est un centre chiral.

10. Réactif de reconnaissance génétique selon la revendication 9, dans lequel le carbone y est un centre chiral.

11. Réactif de reconnaissance génétique selon la revendication 10, dans lequel R₂ est :

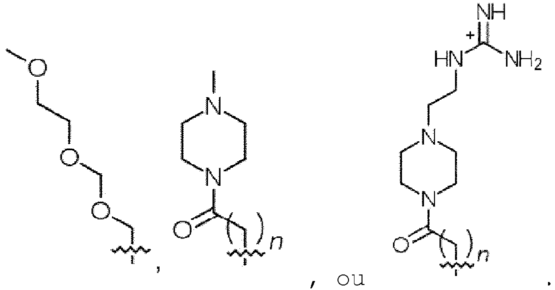

12. Réactif de reconnaissance génétique selon la revendication 9, dans lequel R₁, R₃, R₄, R₅ et R₆ sont H.

13. Réactif de reconnaissance génétique selon l'une quelconque des revendications 1 à 12, dans lequel la pluralité de groupements de bases azotées forment une séquence qui comprend : TTC, TTCTTC, TCT, TCTTCT, CTT, CTTCTT, CCG, CCGCCG, CGC, CGCCGC, GCC, GCCGCC, CGG, CGGCGG, GCG, GCGGCG, GGC, GGCGGC, CTG, CTGCTG, TGC, TGCTGC, GCT, GCTGCT, CAG, CAGCAG, AGC, AGCAGC, GCA, GCAGCA, CAGG, CAGGCAGG, AGGC, AGGCAGGC, GGCA, GGCAGGCA, GCAG, GCAGGCAG, AGAAT, GAATA, AATAG, ATAGA, TAGAA, GGCCCC, GCCCCG, CCCCGG, CCCGGC, CCGGCC, et CGGCCC, ou une répétition contiguë de l'une quelconque des séquences précédentes.

14. Réactif de reconnaissance génétique selon l'une quelconque des revendications 1 à 13, dans lequel la pluralité de groupements de bases azotées est agencée en une séquence complémentaire d'une séquence cible d'un acide nucléique.

15. Réactif de reconnaissance génétique selon l'une quelconque des revendications 1 à 14, comportant de 3 à 25 groupements de bases azotées.
